# EUROPEAN PATENT SPECIFICATION

(11) **EP 0 656 783 B1**
(45) Date of publication and mention of the grant of the patent: **20.11.1996**
(21) Application number: 93916980.1
(22) Date of filing: 02.07.1993
(51) Int. Cl.: A61K 38/00

(54) **PHARMACEUTICALS CONTAINING ONCONASE AND CISPLATIN, MELPHALAN AND ADRIAMYCIN**
ONCONASE UND CISPLATIN, MELPHALAN UND ADRIAMYCIN ENTHALTENDE ARZNEIMITTEL
PRODUITS PHARMACEUTIQUES CONTENANT ONCONASE ET CISPLATINE, MELPHALAN ET ADRIAMYCINE

(30) Priority: 30.07.1992 US 921180
(43) Date of publication of application: 14.06.1995
(73) Proprietor: ALFACELL CORPORATION, Bloomfield NJ 07003 (US)
(72) Inventor: MIKULSKI, Stanislaw, M., Essex Fells, NJ 07021 (US); ARDELT, Wojciech, J., New City, NY 10956 (US)
(74) Representative: Andrae, Steffen, Dr.
(86) International application number: US9306357
(87) International publication number: WO9403197

(56) References cited:
- WO-A-91/07435
- INTERNATIONAL JOURNAL OF ONCOLOGY vol. 1, no. 7 , December 1992 pages 779 - 785 MIKULSKI S. M. ET AL. 'In vitro synergism between a novel amphibian oocytic ribonuclease (ONCONASE) and tamoxifen, lovastatin and cisplatin, in human OVCAR-3 ovarian carcinoma cell line'

## Description

The invention relates to pharmaceuticals, and more particularly relates to pharmaceuticals for use in treating cells which cause cancer tumors in humans.

PCT patent applications WO 89/09606 and WO 91/07435 disclose a pharmaceutical which will be referred to herein by the trademark ONCONASE. It has now been determined that when this pharmaceutical is used in vitro in a combined therapy with three other drugs, the results of the combined therapy are, in certain instances, much more bioactive than would be expected.

These other drugs are known as Cisplatin, Melphalan and Adriamycin.

### DETAILED DESCRIPTION OF PREFERRED EMBODIMENTS

In vitro data indicate that a combination of ONCONASE with a drug sold under the name Cisplatin is much more bioactive against human ovarian OVCAR-3 adenocarcinoma than would be expected, given the separate activities of ONCONASE and Cisplatin. In vitro also data indicate that a combination of ONCONASE with a drug sold under the name Melphalan is much more bioactive against human ovarian OVCAR-3 adenocarcinoma than would be expected, given the separate activities of ONCONASE and Melphalan. In vitro data indicate that a combination of ONCONASE with a drug sold under the name Adriamycin is much more bioactive against human ovarian OVCAR-3 adenocarcinoma than would be expected, given the separate activities of ONCONASE and Adriamycin.

The preferred embodiment of the invention was tested using a cell culture assay. In such an assay, a cell line of known growth rate over a predetermined period is treated with the substance under test and the growth of the treated cells is compared with the growth which would ordinarily be expected from untreated cells.

ONCONASE, described in the above-referenced patent applications and manufactured in accordance with the methodology described in PCT patent application WO 89/09606 was dissolved in phosphate buffered saline (PBS) to obtain 1mg/ml stock solution.

Cisplatin, also known as cis-diamminedichloroplatinum, is a heavy metal complex containing a central atom of platinum surrounded by two chloride atoms and two ammonia molecules in the cis position. It has the molecular formula Pt Cl₂H₆N₂ and a molecular weight of 300.1.

Melphalan, also known as L-phenylalanine mustard, phenylalanine mustard, L-PAM, or L-sarcolysin, is 4-[bis(2-chloroethyl)amino]-L-phenylalanine.

Adriamycin is a trademark for Doxorubicin HCl, USP. Doxorubicin is an anthracycline antibiotic isolated from cultures of Streptomyces peucetius var. caesius. Doxorubicin consists of a napthacenequinone nucleus linked through a glycosidic bond at ring atom 7 to an amino sugar, daunosamine.

The assay system utilized the OVCAR-3 human ovarian adenocarcinoma cell line, obtained from the American Type Culture Collection (accession number ATCC HTB 161). The cell line was cultured in RPMI 1640 medium and supplemented with 20% heat-inactivated fetal bovine serum, 200 mM L-glutamine, 10,000 units per 1 ml penicillin, 10 mg per 1 ml streptomycin, 25 µg per 1 ml fungizone, 10mM HEPES buffer and 10 µg per 1 ml bovine insulin. This was the complete growth medium.

The cells were seeded into 96-well tissue culture plates at a density of 6000 viable cells (50 µl) per well. The cells were allowed to settle for 24 hours and then 50 µl of appropriate ONCONASE and/or Cisplatin, Melphalan or Adriamycin solutions were added per well. The following final concentrations were used:
a) ONCONASE, 20 ng to 10 µg/ml;
b) Cisplatin, 10 to 500 nM;
c) Melphalan, 0.25 to 5 µM; and
d) Adriamycin, 5 to 100 nM.
The plates were incubated for an additional six days at 37 °C and 5% carbon dioxide atmosphere. The total assay time was consequently seven days (one day in which the cells are allowed to settle, and six days of incubation). Percentages of viable cells were then determined using the MTT colorimetric assay using the Bio-Rad EIA microtiter plate reader.

The number of cells was determined by a direct count in an AO-Spencer "Brightline" hemocytometer with a Neubauer ruling. Attached cells were washed three times with Hanks' Balanced Salt Solution and treated with 2 ml of a 0.25% Trypsin - 0.02% EDTA solution in buffered saline for about thirty seconds. The solution was removed and the cells were left at 37 °C for 10 minutes, then suspended in 10 ml of the complete growth medium. The 0.25 ml of the cell suspension was diluted with 0.75 ml of the complete growth medium and then 1 ml of 0.5% Trypan Blue solution was added and viable cells were counted.

Tables 1, 2 and 3 present the result of the above experiments. Except for the ED₅₀ values, these tables are expressed in the Interaction Index developed by Berenbaum. In this Interaction Index, a result of 1.0 indicates that the two drugs do not interact, i.e. that their combined effectiveness against a particular cell line is unchanged from what would be expected from using them individually. An Interaction Index result which is greater than 1.0 indicates that the two drugs are antagonistic, i.e. that their combined effectiveness against a particular cell line is less than what would be expected from using them individually. Progressively higher Interaction Index results indicate progressively greater antagonism. An Interaction Index result which is less than 1.0 indicates that the two drugs are synergistic, i.e. that their combined effectiveness against a particular cell line is greater than what would be expected from using them individually. As the Interaction Index approaches 0 (maximum synergism), this indicates progressively greater synergism. Thus, a lower number indicates a higher bioactivity against the cell line used in the experiment. The ED₅₀ values represent isoeffective doses; they indicate the dose required to halve the growth rate of the cells undergoing the assay. Thus, the lower the ED₅₀ figure, the smaller the dose required to achieve the same bioactivity.

**Table 1**

| Interaction Index and ED₅₀ Values for varying doses of ONCONASE together with Cisplatin for OVCAR-3 human ovarian adenocarcinoma cells and ED₅₀ values for ONCONASE and Cisplatin individually | | | | | |
|---|---|---|---|---|---|
| **ONCONASE Dose (µg/ml)** | **0** | **0.02** | **0.2** | **2.0** | **ED**_{**50**} |
| ONCONASE Alone | | | | | 1.447 |
| ONCONASE + 10 mM Cisplatin | | 1.159 | 0.891 | 0.482 | 0.697 |
| ONCONASE + 25 mM Cisplatin | | 1.098 | 0.830 | 0.421 | 0.609 |
| ONCONASE + 50 mM Cisplatin | | 1.132 | 0.864 | 0.455 | 0.658 |
| ONCONASE + 100 mM Cisplatin | | 0.858 | 0.590 | 0.181 | 0.262 |
| ONCONASE + 250 mM Cisplatin | | 0.700 | 0.432 | 0.023 | 0.034 |
| ONCONASE + 500 mM Cisplatin | | 0.685 | 0.417 | 0.008 | 0.011 |
| ED₅₀ Values*: | 316.509 | 214.299 | 129.616 | <0.001 | |

| | | | | | |
|---|---|---|---|---|---|
| *for Cisplatin in combination with respective concentrations of ONCONASE (vertically); ED₅₀ value for Cisplatin at 0 µl ONCONASE is for Cisplatin alone, OVCAR-3 cells being considered resistant to Cisplatin alone | | | | | |

**Table 2**

| Interaction Index and ED₅₀ Values for varying doses of ONCONASE together with Melphalan for OVCAR-3 human ovarian adenocarcinoma cells and ED₅₀ values for ONCONASE and Melphalan individually | | | | | |
|---|---|---|---|---|---|
| **ONCONASE Dose (µg/ml)** | **0** | **0.02** | **0.2** | **2.0** | **ED**_{**50**} |
| ONCONASE Alone | | | | | 2.317 |
| ONCONASE + 0.25 µM Melphalan | | 1.701 | 1.492 | 0.747 | 1.705 |
| ONCONASE + 0.5 µM Melphalan | | 1.778 | 1.569 | 0.824 | 1.881 |
| ONCONASE + 0.75 µM Melphalan | | 1.746 | 1.537 | 0.792 | 1.808 |
| ONCONASE + 1.0 µM Melphalan | | 1.142 | 0.934 | 0.189 | 0.411 |
| ONCONASE + 2.5 µM Melphalan | | 0.987 | 0.779 | 0.034 | 0.051 |
| ONCONASE + 5.0 µM Melphalan | | 0.975 | 0.766 | 0.021 | 0.022 |
| ED₅₀ Values*: | 4.786 | 4.623 | 3.624 | 0.056 | |

| | | | | | |
|---|---|---|---|---|---|
| *for Melphalan in combination with respective concentrations of ONCONASE (vertically); ED₅₀ value for Melphalan at 0 µl ONCONASE is for Melphalan alone, OVCAR-3 cells being considered resistant to Melphalan alone | | | | | |

**Table 3**

| Interaction Index and ED₅₀ Values for varying doses of ONCONASE together with Adriamycin for OVCAR-3 human ovarian adenocarcinoma cells and ED₅₀ values for ONCONASE and Adriamycin individually | | | | | |
|---|---|---|---|---|---|
| **ONCONASE Dose (µg/ml)** | **0** | **0.02** | **0.2** | **2.0** | **ED**_{**50**} |
| ONCONASE Alone | | | | | 1.166 |
| ONCONASE + 5 nM Adriamycin | | 1.744 | 1.305 | 0.881 | 0.957 |
| ONCONASE + 10 nM Adriamycin | | 1.702 | 1.263 | 0.839 | 0.908 |
| ONCONASE + 25 nM Adriamycin | | 1.208 | 0.769 | 0.346 | 0.332 |
| ONCONASE + 50 nM Adriamycin | | 0.923 | 0.484 | 0.061 | 0.001 |
| ED₅₀ Values*: | 37.340 | 34.473 | 18.090 | 2.265 | |

| | | | | | |
|---|---|---|---|---|---|
| *for Adriamycin in combination with respective concentrations of ONCONASE (vertically); ED₅₀ value for Adriamycin at 0 µl ONCONASE is for Adriamycin alone, OVCAR-3 cells being considered resistant to Adriamycin alone | | | | | |

These results demonstrate that, in the instances shown, the bioactivities of ONCONASE combined with Cisplatin, Melphalan and Adriamycin on OVCAR-3 human ovarian adenocarcinoma are much greater than would be expected from the bioactivities of the individual drugs alone. (Indeed, the OVCAR-3 cell line originated from a patient who was clinically resistant to Cisplatin, Melphalan and Adriamycin.) This may be seen from the ED₅₀ figures which are along the right edge of the Tables. These figures represent computed isoeffective doses; the figure shown is the amount of material which would be required to halve the growth rate of the cells undergoing the assay. Thus, the lower the ED₅₀ figure, the smaller the dose required to achieve the same bioactivity.

It will be understood that ONCONASE, on the one hand, and Cisplatin, Melphalan or Adriamycin, on the other hand, need not be (and indeed usually will not be) simultaneously or contemporaneously administered to the patient. Rather, the ONCONASE may be administered according to one dose schedule and the Cisplatin, Melphalan or Adriamycin administered according to another dose schedule. It is only necessary that the doses and schedules be so chosen that the ONCONASE and the Cisplatin, Melphalan or Adriamycin interact within the body of the patient.

### Chemical Analysis and Composition of ONCONASE

ONCONASE has been well characterized chemically. While ONCONASE is a protein isolated from rana pipiens, it is believed that ONCONASE may be produced using genetic engineering techniques, as long as the end result has the following chemistry and structure:

ONCONASE is a pure protein (i.e. homogeneous, as established by standard tests which are used to assay the homogeneity of proteins). By electrophoresis, the molecular weight of ONCONASE is approximately 14,500 Daltons. Calculation of the molecular weight based upon the below listed amino acid sequence indicates that the molecular weight should be 11,819 Daltons. However, because metal ions may have bonded to the protein despite all efforts to remove them, and because different isotopes may be involved, the molecular weight of ONCONASE as determined by mass spectroscopy is 12,430 Daltons. In view of this discrepancy, the molecular weight of ONCONASE as determined by mass spectrometry will be considered to be approximately 12,000 Daltons. ONCONASE has an isoelectric point pI which is at least 9.5 and may be as high as 10.5. ONCONASE has a blocked amino terminal group and is essentially free of carbohydrates (as determined by anthrone and orcinol methods).

### ONCONASE has the following amino acid composition:

| Amino Acid Analysis | |
|---|---|
| AMINO ACID RESIDUE | MOL % (24 HOUR ACID HYDROLYSIS) |
| Aspartic acid/Asparagine | 13.39 |
| Threonine | 9.84 (Note 1) |
| Serine | 8.08 (Note 1) |
| Glutamic acid/Glutamine | 5.88 |
| Proline | 3.98 |
| Glycine | 2.98 |
| Alanine | 2.92 |
| Cystine/2 | 7.77 |
| Valine | 7.77 |
| Methionine | 0.94 |
| Isoleucine | 5.29 (Note 2) |
| Leucine | 4.95 |
| Tyrosine | 2.85 |
| Phenylalanine | 5.73 |
| Histidine | 2.99 |
| Lysine | 11.78 |
| Arginine | 2.85 |
| Tryptophan | Not Determined (Note 3) |
| Approximate Total | 99.99 % |
| Note 1: Threonine and serine are partially destroyed during hydrolysis and this value is corrected for such partial destruction. Note 2: This value is corrected for incomplete hydrolysis. Note 3: Tryptophan cannot be detected in acid hydrolysis of proteins because it is destroyed and is consequently shown as Not Determined. However, analysis of the ultraviolet spectrum revealed the presence of one tryptophan residue per molecule. | |

| Amino Acid Composition (as calculated from amino acid sequence) | |
|---|---|
| AMINO ACID | NUMBER OF RESIDUES PER MOLECULE OF MATERIAL |
| Aspartic acid | 6 |
| Asparagine | 8 |
| Threonine | 10 |
| Serine | 8 |
| Glutamic acid | 3 |
| Pyroglutamic acid | 1 |
| Glutamine | 2 |
| Proline | 4 |
| Glycine | 3 |
| Alanine | 3 |
| Cystine/2 | 8 |
| Valine | 8 |
| Methionine | 1 |
| Isoleucine | 6 |
| Leucine | 5 |
| Tyrosine | 3 |
| Phenylalanine | 6 |
| Histidine | 3 |
| Lysine | 12 |
| Arginine | 3 |
| Tryptophan | 1 |
| Approximate Total | 104 |

ONCONASE has been sequenced. As is shown below, the total length of the sequence is 104 residues. The N-terminus of the protein is pyroglutamic acid (<Glu). This is a cyclized derivative of glutamic acid which is devoid of the free amino group necessary for direct sequencing and which therefore "blocks" the N-terminus of the protein.

When the shorter fragment described in PCT patent application WO 89/09606 was cleaved with pyroglutamate aminopeptidase, pyroglutamic acid was removed from the shorter fragment, permitting sequencing to commence at the second residue. Such cleavage is a strong indication that the N-terminus is pyroglutamic acid since pyroglutamate aminopeptidase only cleaves pyroglutamic acid. The presence of pyroglutamic acid was further confirmed by mass spectrometry of the referenced shorter fragment. The molecular weight of this shorter fragment determined by mass spectrometry agreed well with the weight as calculated assuming that pyroglutamic acid was present and disagreed with the weight as calculated assuming that glutamic acid was present.

ONCONASE has the following amino acid sequence:

1 2 3 4 5 6 7 8 9 10
<Glu-Asp-Trp-Leu-Thr-Phe-Gln-Lys-Lys-His-

11 20
Ile-Thr-Asn-Thr-Arg-Asp-Val-Asp-Cys-Asp-

21 30
Asn-Ile-Met-Ser-Thr-Asn-Leu-Phe-His-Cys-

31 40
Lys-Asp-Lys-Asn-Thr-Phe-Ile-Tyr-Ser-Arg-

41 50
Pro-Glu-Pro-Val-Lys-Ala-Ile-Cys-Lys-Gly-

51 60
Ile-Ile-Ala-Ser-Lys-Asn-Val-Leu-Thr-Thr-

61 70
Ser-Glu-Phe-Tyr-Leu-Ser-Asp-Cys-Asn-Val-

71 80
Thr-Ser-Arg-Pro-Cys-Lys-Tyr-Lys-Leu-Lys-

81 90
Lys-Ser-Thr-Asn-Lys-Phe-Cys-Val-Thr-Cys-

91 100
Glu-Asn-Gln-Ala-Pro-Val-His-Phe-Val-Gly-

101 104
Val-Gly-Ser-Cys

Although a preferred embodiment has been described above, the scope of the invention is limited only by the following claims:

## Claims

1. A pharmaceutical composition comprising
a protein having a molecular weight of approximately 12,000 Daltons by mass spectrometry or approximately 14,500 Daltons by gel electrophoresis, and an amino acid composition approximately as follows:
| AMINO ACID RESIDUE | MOL % (24 HOUR ACID HYDROLYSIS) |
|---|---|
| Aspartic acid/Asparagine | 13.39 |
| Threonine | 9.84 |
| Serine | 8.08 |
| Glutamic acid/Glutamine | 5.88 |
| Proline | 3.98 |
| Glycine | 2.98 |
| Alanine | 2.92 |
| Cystine/2 | 7.77 |
| Valine | 7.77 |
| Methionine | 0.94 |
| Isoleucine | 5.29 |
| Leucine | 4.95 |
| Tyrosine | 2.85 |
| Phenylalanine | 5.73 |
| Histidine | 2.99 |
| Lysine | 11.78 |
| Arginine | 2.85 |
| Tryptophan | Not Determined |
| Approximate Total | 99.99 % |
and either
a) Cisplatin,
b) 4-[bis(2-chloroethyl)amino]-L-phenylalanine,
or
c) Doxorubicin HCl, USP.

2. A pharmaceutical composition comprising
a protein having a molecular weight of approximately 12,000 Daltons by mass spectrometry or approximately 14,500 Daltons by gel electrophoresis, and an amino acid composition approximately as follows:
| AMINO ACID | NUMBER OF RESIDUES PER MOLECULE OF MATERIAL |
|---|---|
| Aspartic acid | 6 |
| Asparagine | 8 |
| Threonine | 10 |
| Serine | 8 |
| Glutamic acid | 3 |
| Pyroglutamic acid | 1 |
| Glutamine | 2 |
| Proline | 4 |
| Glycine | 3 |
| Alanine | 3 |
| Cystine/2 | 8 |
| Valine | 8 |
| Methionine | 1 |
| Isoleucine | 6 |
| Leucine | 5 |
| Tyrosine | 3 |
| Phenylalanine | 6 |
| Histidine | 3 |
| Lysine | 12 |
| Arginine | 3 |
| Tryptophan | 1 |
| Approximate Total | 104 |
and either
a) Cisplatin,
b) 4-[bis(2-chloroethyl)amino]-L-phenylalanine, or
c) Doxorubicin HCl, USP.

3. The pharmaceutical composition of claim 1 or claim 2, wherein the protein contained therein is a pure protein having an isoelectric point pI which is at least 9.5 and having a blocked amino terminal group, the protein further being essentially free of carbohydrates.

4. The composition of claim 3, wherein the predominant amino acids of the protein are lysine and threonine.

5. The composition of claim 3, wherein each molecule of the protein contains one residue of tryptophan.

6. The composition of claim 3, wherein each molecule of the protein contains one residue of methionine.

7. A pharmaceutical composition comprising a protein having the following amino acid sequence: 
1         2         3         4         5         6         7         8         9         10
<Glu-Asp-Trp-Leu-Thr-Phe-Gln-Lys-Lys-His-

11                                                                                    20
Ile-Thr-Asn-Thr-Arg-Asp-Val-Asp-Cys-Asp-

21                                                                                    30
Asn-Ile-Met-Ser-Thr-Asn-Leu-Phe-His-Cys-

31                                                                                    40
Lys-Asp-Lys-Asn-Thr-Phe-Ile-Tyr-Ser-Arg-

41                                                                                    50
Pro-Glu-Pro-Val-Lys-Ala-Ile-Cys-Lys-Gly-

51                                                                                    60
Ile-Ile-Ala-Ser-Lys-Asn-Val-Leu-Thr-Thr-

61                                                                                    70
Ser-Glu-Phe-Tyr-Leu-Ser-Asp-Cys-Asn-Val-

71                                                                                    80
Thr-Ser-Arg-Pro-Cys-Lys-Tyr-Lys-Leu-Lys-

81                                                                                    90
Lys-Ser-Thr-Asn-Lys-Phe-Cys-Val-Thr-Cys-

91                                                                                    100
Glu-Asn-Gln-Ala-Pro-Val-His-Phe-Val-Gly-

101              104
Val-Gly-Ser-Cys
 and a drug selected from the group consisting of
a) Cisplatin,
b) 4-[bis(2-chloroethyl)amino]-L-phenylalanine,
and
c) Doxorubicin HCl, USP.

## Patentansprüche

1. Pharmazeutische Zusammensetzung mit
einem Protein mit einem Molekulargewicht von etwa 12.000 Dalton, bestimmt durch Massenspektrometrie, oder etwa 14.500 Dalton, bestimmt durch Gelelektrophorese, und einer Aminosäurezusammensetzung, die annähernd wie folgt ist:
| Aminosäurerest | Mol % (24-stündige saure Hydrolyse) |
|---|---|
| Asparaginsäure/Asparagin | 13,39 |
| Threonin | 9,84 |
| Serin | 8,08 |
| Glutaminsäure/Glutamin | 5,88 |
| Prolin | 3,98 |
| Glycin | 2,98 |
| Alanin | 2,92 |
| Cystin/2 | 7,77 |
| Valin | 7,77 |
| Methionin | 0,94 |
| Isoleucin | 5,29 |
| Leucin | 4,95 |
| Tyrosin | 2,85 |
| Phenylalanin | 5,73 |
| Histidin | 2,99 |
| Lysin | 11,78 |
| Arginin | 2,85 |
| Tryptophan | nicht bestimmt |
| Näherungssumme | 99,99 % |
sowie entweder
a) Cisplatin,
b) 4-[Bis(2-chlorethyl)amino]-L-phenylalanin, oder
c) Doxorubicin HCl, USP.

2. Pharmazeutische Zusammensetzung mit
einem Protein mit einem Molekulargewicht von etwa 12.000 Dalton, bestimmt durch Massenspektrometrie, oder etwa 14.500 Dalton, bestimmt durch Gelelektrophorese, und einer Aminosäurezusammensetzung, die annähernd wie folgt ist:
| Aminosäure | Anzahl an Resten pro Molekül des Materials |
|---|---|
| Asparaginsäure | 6 |
| Asparagin | 8 |
| Threonin | 10 |
| Serin | 8 |
| Glutaminsäure | 3 |
| Pyroglutaminsäure | 1 |
| Glutamin | 2 |
| Prolin | 4 |
| Glycin | 3 |
| Alanin | 3 |
| Cystin/2 | 8 |
| Valin | 8 |
| Methionin | 1 |
| Isoleucin | 6 |
| Leucin | 5 |
| Tyrosin | 3 |
| Phenylalanin | 6 |
| Histidin | 3 |
| Lysin | 12 |
| Arginin | 3 |
| Tryptophan | 1 |
| Näherungssumme | 104 |
sowie entweder
a) Cisplatin,
b) 4-[Bis(2-chlorethyl)amino]-L-phenylalanin,
oder
c) Doxorubicin HCl, USP.

3. Pharmazeutische Zusammensetzung nach Anspruch 1 oder Anspruch 2, bei der das darin enthaltene Protein ein reines Protein mit einem isoelektrischen Punkt pI ist, der wenigstens 9,5 beträgt, und das eine blockierte terminale Aminogruppe aufweist, wobei das Protein außerdem im wesentlichen frei von Kohlenhydraten ist.

4. Zusammensetzung nach Anspruch 3, bei der die überwiegenden Aminosäuren des Proteins Lysin und Threonin sind.

5. Zusammensetzung nach Anspruch 3, bei der jedes Molekül des Proteins einen Tryptophanrest enthält.

6. Zusammensetzung nach Anspruch 3, bei der jedes Molekül des Proteins einen Methioninrest enthält.

7. Eine pharmazeutische Zusammensetzung mit einem Protein mit der folgenden Aminosäuresequenz: 
1 2 3 4 5 6 7 8 9 10
<Glu-Asp-Trp-Leu-Thr-Phe-Gln-Lys-Lys-His-

11 20
Ile-Thr-Asn-Thr-Arg-Asp-Val-Asp-Cys-Asp-

21 30
Asn-Ile-Met-Ser-Thr-Asn-Leu-Phe-His-Cys-

31 40
Lys-Asp-Lys-Asn-Thr-Phe-Ile-Tyr-Ser-Arg-

41 50
Pro-Glu-Pro-Val-Lys-Ala-Ile-Cys-Lys-Gly-

51 60
Ile-Ile-Ala-Ser-Lys-Asn-Val-Leu-Thr-Thr-

61 70
Ser-Glu-Phe-Tyr-Leu-Ser-Asp-Cys-Asn-Val-

71 80
Thr-Ser-Arg-Pro-Cys-Lys-Tyr-Lys-Leu-Lys-

81 90
Lys-Ser-Thr-Asn-Lys-Phe-Cys-Val-Thr-Cys-

91 100
Glu-Asn-Gln-Ala-Pro-Val-His-Phe-Val-Gly-

101 104
Val-Gly-Ser-Cys
 sowie einem Arzneistoff, der aus der Gruppe ausgewählt ist, die besteht aus
a) Cisplatin,
b) 4-[Bis(2-chlorethyl)amino]-L-phenylalanin, und
c) Doxorubicin HCl, USP.

## Revendications

1. Composition pharmaceutique comprenant
une protéine présentant un poids moléculaire d'approximativement 12000 Daltons par spectrométrie de masse, ou d'approximativement 14500 Daltons par électrophorèse sur gel, et une composition en acides aminés approximativement comme suit:
| Résidu d'acide aminé | % molaire (24 h d'hydrolyse acide) |
|---|---|
| Acide aspartique/asparagine | 13,39 |
| Thréonine | 9,84 |
| Sérine | 8,08 |
| Acide Glutamique/glutamine | 5,88 |
| Proline | 3,98 |
| Glycine | 2,98 |
| Alanine | 2,92 |
| Cystine/2 | 7,77 |
| Valine | 7,77 |
| Méthionine | 0,94 |
| Isoleucine | 5,29 |
| Leucine | 4,95 |
| Tyrosine | 2,85 |
| Phénylalanine | 5,73 |
| Histidine | 2,99 |
| Lysine | 11,78 |
| Arginine | 2,85 |
| Tryptophane | non déterminé |
| Total approximatif | 99,99 % |
et soit
a) Cisplatine,
b) 4-[bis-2-chloroéthyl)amino]-L-phénylalanine, soit
c) Doxorubicine HCl, USP.

2. Composition pharmaceutique comprenant
une protéine présentant un poids moléculaire d'approximativement 12000 Daltons par spectrométrie de masse, ou d'approximativement 14500 Daltons par électrophorèse sur gel, et une composition en acides aminés approximativement comme suit:
| Acide aminé | nombre de résidus par molécule du matériau |
|---|---|
| Acide aspartique | 6 |
| Asparagine | 8 |
| Thréonine | 10 |
| Sérine | 8 |
| Acide glutamique | 3 |
| Acide pyroglutamine | 1 |
| Glutamine | 2 |
| Proline | 4 |
| Glycine | 3 |
| Alanine | 3 |
| Cystine/2 | 8 |
| Valine | 8 |
| Méthionine | 1 |
| Isoleucine | 6 |
| Leucine | 5 |
| Tyrosine | 3 |
| Phénylalanine | 6 |
| Histidine | 3 |
| Lysine | 12 |
| Arginine | 3 |
| Tryptophane | 1 |
| Total approximatif | 104 |
et soit
a) Cisplatine,
b) 4-[bis-2-chloroéthyl)amino]-L-phénylalanine, soit
c) Doxorubicine HCl, USP.

3. Composition pharmaceutique selon la revendication 1 ou la revendication 2, dans laquelle la protéine qu'elle contient est une protéine pure présentant un point isoélectrique pI qui est d'au moins 9,5 et présentant un groupe terminal amino bloqué, la protéine étant de plus essentiellement exempte d'hydrates de carbone.

4. Composition selon la revendication 3, dans laquelle les acides aminés prédominants de la protéine sont la lysine et la thréonine.

5. Composition selon la revendication 3, dans laquelle chaque molécule de la protéine contient un résidu de tryptophane.

6. Composition selon la revendication 3, dans laquelle la molécule de la protéine contient un résidu de méthionine.

7. Composition pharmaceutique comprenant une protéine présentant la séquence suivante d'acides aminés: 
1       2       3       4       5       6       7       8       9       10
<Glu-Asp-Trp-Leu-Thr-Phe-Gln-Lys-Lys-His-

11                                                                      20
Ile-Thr-Asn-Thr-Arg-Asp-Val-Asp-Cys-Asp-

21                                                                      30
Asn-Ile-Met-Ser-Thr-Asn-Leu-Phe-His-Cys-

31                                                                      40
Lys-Asp-Lys-Asn-Thr-Phe-Ile-Tyr-Ser-Arg-

41                                                                      50
Pro-Glu-Pro-Val-Lys-Ala-Ile-Cys-Lys-Gly-

51                                                                      60
Ile-Ile-Ala-Ser-Lys-Asn-Val-Leu-Thr-Thr-

61                                                                      70
Ser-Glu-Phe-Tyr-Leu-Ser-Asp-Cys-Asn-Val-

71                                                                      80
Thr-Ser-Arg-Pro-Cys-Lys-Tyr-Lys-Leu-Lys-

81                                                                      90
Lys-Ser-Thr-Asn-Lys-Phe-Cys-Val-Thr-Cys-

91                                                                      100
Glu-Asn-Gln-Ala-Pro-Val-His-Phe-Val-Gly-

101             104
Val-Gly-Ser-Cys
 et un médicament choisi dans le groupe formé de
a) Cisplatine,
b) 4-[bis-2-chloroéthyl)amino]-L-Phénylalanine, et
c) Doxorubicine HCl, USP.
